# DEMANDE DE BREVET EUROPEEN

(11) **EP 1 449 518 A1**
(43) Date de publication de la demande: **25.08.2004**
(21) Numéro de dépôt: 04300077.7
(22) Date de dépôt: 12.02.2004
(51) Int. Cl.: A61K 7/48, A61K 31/198, A61P 3/04

(54) **Utilisation de N-octanoyl aminoacides comme actif cosmétique et pharmaceutique amincissant**

(30) Priorité: 21.02.2003 FR 0302162
(71) Demandeur: SOCIETE D'EXPLOITATION DE PRODUITS POUR LES INDUSTRIES CHIMIQUES, S.E.P.P.I.C., 75321 Paris Cédex 07 (FR)
(72) Inventeur: Garcia, Christine, 81100 Castres (FR)
(74) Mandataire: Conan, Philippe Claude

(57) **Abrégé**

Utilisation d'un composé de formule (I) : 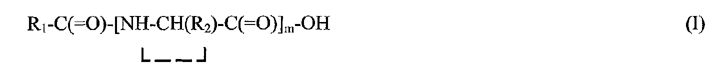 dans laquelle R₁ représente un radical hydrocarboné comportant 7 atomes de carbone, R₂ représente la chaîne caractérisante d'un acide aminé et m est compris entre 1 et 50, ou d'un mélange desdits composés de formule (I), comme actif amincissant. Procédé de traitement non thérapeutique mettant en oeuvre ledit composé et utilisation dudit composé pour préparer un médicament à activité lipolytique, destiné à induire l'amincissement du corps humain.

## Description

La présente invention a pour objet une nouvelle utilisation d'actifs cosmétiques pour amincir le corps humain.

Une partie de la graisse du corps humain est stockée sous forme de triglycérides, dans des cellules du tissu gras du derme, appelés adipocytes. L'amincissement rend compte d'une diminution de la graisse stockée dans les adipocytes. Ce processus nécessite une étape prioritaire qui a lieu à l'intérieur de ces cellules et qui consiste en l'hydrolyse des triglycérides en acides gras et glycérol. Ce phénomène s'appelle la lipolyse.

La plupart des formulations cosmétiques amincissantes commercialisées aujourd'hui, contiennent au moins un composé possédant une activité lipolytique. Le plus fréquemment utilisé est la caféine mais la théophylline est aussi connue pour posséder une telle propriété.

A l'occasion de leur recherche de nouveaux actifs à activité lipolytique, qui aient une meilleure compatibilité avec la peau que ceux de l'état de la technique, les inventeurs ont mis en évidence que certains dérivés N-acylés d'acides aminés connus pour leur propriété apaisante possédaient aussi une propriété lipolytique plus efficace que celle de la caféine

C'est pourquoi, selon un premier aspect, l'invention a pour objet, l'utilisation d'un composé de formule (1) : 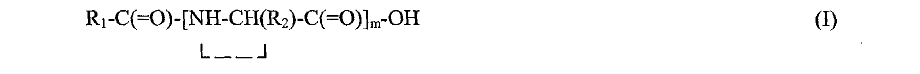 dans laquelle R₁ représente un radical hydrocarboné aliphatique, saturé ou insaturé, linéaire ou ramifié, comportant 7 atomes de carbone, R₂ représente la chaîne caractérisante d'un acide aminé et m est compris entre 1 et 50, ou d'un mélange desdits composés de formule (I), comme actif amincissant dans une composition contenant un milieu cosmétiquement acceptable.

Le composé de formule (I) telle que définie ci-dessus, peut être sous forme d'acide libre ou sous forme partiellement ou totalement salifiée. Lorsque le composé de formule (I) est sous forme salifiée, il s'agit notamment de sels alcalins tels que les sels de sodium, de potassium ou de lithium, de sels alcalino-terreux tels que les sels de calcium, de magnésium ou de strontium ; de sel d'ammonium ou de sel d'un aminoalcool comme le sel de (2-hydroxy éthyl) ammonium. Il peut aussi s'agir de sels métalliques tels que les sels divalents de zinc ou de manganèse, les sels trivalents de fer, de lanthane, de cérium ou d'aluminium. De façon générale, le taux de salification du composé de formule (I) telle que définie ci dessus, dépendra en autre de son pK_{A} et de la concentration en sels de la composition dans laquelle il est incorporé, le composé de formule (I) sera présent.

Dans l'exposé suivant, par composé de formule (I), on entend composé de formule (I) sous forme libre ou sous forme partiellement ou totalement salifiée.

L'expression "chaîne caractérisante" utilisée pour définir le radical R₂, désigne la chaîne principale non fonctionnelle de l'acide aminé considéré. Ainsi, pour un acide aminé représenté par la formule générale (IIIa) :

H₂N-CH(R₂)-C(=O)-OH (IIIa),

comme pour un acide aminé cyclique représenté par la formule (IIIb) : 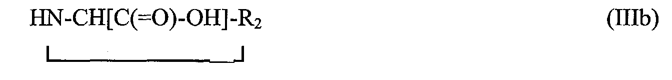 la chaîne caractérisante sera la chaîne représentée par R₂.

R₂ représente notamment la chaîne caractérisante d'un acide aminé choisi parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la cystine, la méthionine, l'hydroxy proline, l'hydroxy lysine, la sarcosine ou l'omithine.

L'invention a principalement pour objet, l'utilisation d'un composé de formule (I) telle que définie précédemment, dans laquelle, dans le reste

- HN-CH(R₂)-C(=O)- (IIIa),

R₂ représente la chaîne caractérisante de la glycine.

L'invention a plus particulièrement pour objet, l'utilisation d'un composé de formule (I), telle que définie précédemment dans laquelle m est un nombre décimal compris entre 1 et 10 et il est de préférence inférieur à 5.

Selon un aspect tout particulier de la présente invention, dans la formule (I) telle que définie précédemment, m inférieur ou égal à 2 et est plus particulièrement inférieur ou égal à 1,4.

Selon un autre aspect tout particulier de la présente invention, dans la formule (I) telle que définie précédemment, m égal à 1.

Selon une autre variante particulière de la présente invention, on utilise un seul composé de formule (I), telle que définie précédemment, dans la composition contenant le milieu cosmétiquement acceptable.

Selon une autre variante particulière de la présente invention, on utilise un mélange de composés de formule (I) telle que définie précédemment.

Les composés de formules (I) sont généralement obtenus par N-acylation de composés de formules (IIIa) ou (IIIb), telles que définies précédemment ou de leurs sels.

Lorsqu'il s'agit d'un mélange de composés de formules (I), il est par exemple obtenu par N-acylation du mélange d'acides aminés résultant de l'hydrolyse totale ou partielle de protéines de toutes origines.

Ces protéines peuvent être d'origine animale, telles que, par exemple, le collagène, l'élastine, la protéine de chair de poisson, la gélatine de poissons, la kératine ou la caséine, d'origine végétale, comme les protéines de céréales, de fleurs ou de fruits, telles que par exemple, les protéines issues du soja, du tournesol, de l'avoine, du blé, du maïs, de l'orge, de la pomme de terre, du lupin, de la féverole, de l'amande douce, du kiwi, de la mangue ou de la pomme ; il peut s'agir aussi de protéines obtenues à partir de chorelles (algues unicellulaires), d'algues roses, de levures ou de la soie.

Cette hydrolyse est réalisée par exemple, par chauffage à des températures comprises entre 60°C et 130°C d'une protéine placée dans un milieu acide ou alcalin.

Cette hydrolyse peut également être réalisée par voie enzymatique avec une protéase, couplée éventuellement à une post-hydrolyse alcaline ou acide. Quand m est supérieur à 1, R₂ représente une seule même chaîne ou bien plusieurs chaînes caractérisant différents acides aminés, selon la protéine hydrolysée et le degré d'hydrolyse.

Les aminogrammes de quelques protéines d'origine végétale sont consignés dans les tableaux suivants :

**Tableau 1**

| | Origine de la protéine (proportions en acides aminés exprimées en % pondéraux) | | | |
|---|---|---|---|---|
| | Avoine | Soja | Blé | Tournesol |
| Glycine | 6,9 | 4,2 | 3,2 | 6,2 |
| Alanine | 5,9 | 4,2 | 2,6 | 4,8 |
| Serine | 5,6 | 5,1 | 1,7 | 5,1 |
| Acide aspartique | 16,2 | 11,7 | 3,4 | 10,6 |
| Acide glutamique | 28,3 | 19,1 | 37,9 | 23,6 |
| Valine | 2,9 | 5,0 | 4,2 | 4,8 |
| Thréonine | 3,1 | 3,9 | 2,7 | 4,4 |
| Arginine | 6,6 | 7,8 | 3,7 | 8,4 |
| Lysine | 3,6 | 6,2 | 1,9 | 3,2 |
| Proline | 4,7 | 5,4 | 11,7 | 3,0 |
| Leucine | 6,4 | 8,1 | 7,1 | 6,4 |
| Phénylalanine | 1,4 | 5,0 | 5,4 | 4,3 |
| Isoleucine | 2,2 | 4,8 | 3,7 | 4,1 |
| Histidine | 1,7 | 2,6 | 2,4 | 2,0 |
| Tyrosine | 1,5 | 3,5 | 3,1 | 2,7 |
| Méthionine | 1,2 | 1,2 | 1,6 | 1,8 |
| Cystéine / Cystine | 1,9 | 1,5 | 1,9 | 1,9 |
| Tryptophane | - | 1,0 | 1,0 | 1,3 |

**Tableau 2**

| | Origine de la protéine (proportions en acides aminés exprimées en % pondéraux) | | | |
|---|---|---|---|---|
| | Lupin | Pomme de terre | Féverole | Maïs |
| Glycine | 0,9 | 4,8 | 4,0 | 2,4 |
| Alanine | 2,4 | 5,0 | 4,0 | 7,95 |
| Serine | 6,1 | 5,8 | 4,9 | 5,1 |
| Acide aspartique | 15,8 | 12,5 | 10,5 | 10,6 |
| Acide glutamique | 8,0 | 11,5 | 16,8 | 23,6 |
| Valine | 7,9 | 7,1 | 4,5 | 4,8 |
| Thréonine | 8,1 | 6,1 | 3,6 | 4,4 |
| Arginine | 16,1 | 5,0 | 9,21 | 8,4 |
| Lysine | 7,1 | 7,8 | 6,5 | 6,2 |
| Proline | - | 5,1 | 4,4 | 3,0 |
| Leucine | 7,45 | 10,4 | 7,4 | 8,1 |
| Phénylalanine | 8,6 | 6,4 | 4,4 | 4,3 |
| Isoleucine | 8,7 | 6,1 | 3,9 | 4,1 |
| Histidine | - | 2,2 | 2,6 | 2,0 |
| Tyrosine | - | 5,7 | 3,6 | 2,7 |
| Méthionine | 0,6 | 2,4 | 0,8 | 1,8 |
| Cystéine / Cystine | - | 1,6 | 1,7 | 1,9 |
| Tryptophane | 1,2 | 1,4 | 1,2 | 1,3 |
| Omithine | 0,4 | - | - | - |

La réaction d'acylation est connue de l'homme du métier. Elle est décrite par exemple dans la demande internationale publiée sous le numéro WO 98/09611. Elle est mise en oeuvre indifféremment sur un acide aminé ou sur un mélange d'acides aminés. L'agent d'acylation consiste généralement en un dérivé activé d'un acide carboxylique de formule R₁-C(=O)-OH, dans laquelle R₁ est tel que défini précédemment, tel qu'un anhydride symétrique de cet acide, l'ester méthylique de cet acide, ou un halogénure d'acide comme le chlorure d'acide ou le bromure d'acide.

L'invention a aussi pour objet, un procédé de traitement non thérapeutique du corps humain destiné à l'amincir, caractérisé en ce que l'on y applique une composition contenant un milieu cosmétiquement acceptable et une quantité efficace d'au moins un composé de formule (I) telle que définie précédemment.

L'invention a aussi pour objet l'utilisation d'au moins un composé de formule (I), telle que définie précédemment, pour préparer un médicament à activité lipolytique, destiné à induire l'amincissement du corps humain.

Dans les compositions définies ci-dessus, le composé de formule (I), est généralement mis en oeuvre en une quantité comprise entre 0,01 % et 10 % de leur poids, plus particulièrement entre 0,1 % à 5 % de leurs poids, et tout particulièrement entre 1 % et 5 % de leurs poids.

Comme le montrent les exemples suivants, les composés mis en oeuvre dans les traitements cosmétiques ou thérapeutiques définis précédemment, se caractérisent de façon inattendue, par une activité lipolytique supérieure aux compositions de l'état de la technique. Ils sont donc de façon générale, appropriés aux traitements amincissants du corps humain.

Les compositions mises en oeuvre dans lesdits traitements, se présentent généralement sous forme de solutions aqueuses ou hydroalcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

Les compositions mises en oeuvre dans lesdits traitements, sont administrées au sujet sous les formes classiques utilisées en cosmétique et en pharmacie ; il s'agit plus particulièrement des administrations topique, orale ou parentérale.

De façon générale, les composés de formule (I), sont associés à de nombreux types d'adjuvants ou de principes actifs utilisés dans les formulations cosmétiques, qu'il s'agisse, de corps gras, de solvants organiques, d'épaississants, de gélifiants, d'adoucissants, d'antioxydants, d'opacifiants, de stabilisants, de moussants, de parfums, d'émulsionnants, ioniques ou non, de charges, de séquestrants, de chélateurs, de conservateurs, de filtres chimiques ou de filtres minéraux, d'huiles essentielles, de matières colorantes, de pigments, d'actifs hydrophiles ou lipophiles, d'humectants, par exemple la glycérine, de conservateurs, de colorants, de parfums, d'actifs cosmétiques, de filtres solaires minéraux ou organiques, de charges minérales comme les oxydes de fer, oxydes de titane et le talc, de charges synthétiques comme les nylons et les poly(méthacrylate de méthyle) réticulés ou non, d'élastomères silicone, de séricites ou d'extraits de plantes ou encore de vésicules lipidiques ou tout autre ingrédient habituellement utilisé en cosmétique.

Comme exemples d'huiles que l'on peut associer au composé de formule (I), on peut citer, les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associer à cet actif, on peut citer les alcools gras ou les acides gras.

Parmi les polymères épaississants et/ou émulsionnant utilisés dans la présente invention, il y a par exemple, les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes. Parmi les polymères de type polyélectrolytes, pouvant être mis en jeu dans la production d'une phase aqueuse gélifiée apte à être utilisée dans la préparation d'émulsions E/H contenant les composés de formule (I) objets de la présente invention, il y a par exemple, les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propane sulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL^{TM} EG, SEPIGEL^{TM} 305, SIMULGEL^{TM} NS, SIMULGEL^{TM} 800 et SIMULGEL^{TM} A par la demanderesse.

Parmi les cires utilisables dans la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.
Parmi les émulsionnants utilisables dans la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de principe actif que l'on peut associer au composé de formule (I), on peut citer les composés ayant une action éclaircissante ou dépigmentante tels que par exemple l'arbutine, l'acide kojique, l'hydroquinone, l'acide ellagique, la vitamine C, le magnésium ascorbyl phosphate les extraits de polyphénols, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol) , l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine^{TM}, les extraits de blé par exemple la Tensine^{TM} ou la Gliadine^{TM}, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, les actifs ayant une activité anti-microbienne ou une action purifiante vis à vis des peaux grasses tels que le LIPACIDE^{TM} PVB, les actifs ayant une propriété énergisante ou stimulante comme le SEPITONIC^{TM} M3 ou le Physiogényl^{TM} le panthénol et ses dérivés comme le SEPICAP^{TM} MP, les actifs anti-age comme le SEPILIFT^{TM} DPHP, le LIPACIDE^{TM} PVB, le SEPIVINOL^{TM}, le SEPIVITAL^{TM}, les actifs hydratants comme le SEPICALM^{TM} S, le SEPICALM^{TM} VG et le LIPACIDE^{TM} DPHP, les actifs anti-age " anti-photo vieillissement ", les actifs protecteurs de l'intégrité de la jonction dermo-épidermique, les actifs augmentant la synthèse des composants de la matrice extracellulaire, les actifs ayant une activité amincissante, raffermissante ou drainante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule , des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

Comme filtre solaire que l'on peut incorporer dans la composition selon l'invention, on peut citer tous ceux figurant dans la directive cosmétique 76/768/CEE modifiée annexe VII.

L'étude expérimentale suivante illustre l'invention sans toutefois la limiter.

### A - Evaluation in vitro de l'activité lipolytique des composés de formule (I)

### (1) - But et principe de la méthode

L'expérience à pour objet de mettre en évidence, dans un modèle in vitro d'adipocytes humains isolés, l'activité lipolytique des composés mis en oeuvre. En effet, les triglycérides sont stockés dans les adipocytes et constituent la réserve graisseuse. Pour que cette réserve soit diminuée, ce qui est la finalité recherchée lorsque l'on utilise des produits amincissants, il faut hydrolyser les triglycérides pour permettre l'élimination des acides gras de la cellule. L'hydrolyse des triglycérides en acides gras non estérifiés et en glycérol est dénommée la lipolyse.

La méthode décrite consiste en une incubation des produits en présence d'adipocytes humains en suspension, suivie de la mesure du taux d'acides gras non estérifiés dans le milieu d'incubation des adipocytes. Le taux d'acides gras libres reflète ainsi l'hydrolyse des triglycérides présents dans les adipocytes.

### (2) - Protocole expérimental

### (i) Modèle cellulaire :

Le test est réalisé à partir d'adipocytes humains isolés et préparés en suspension cellulaire. Les adipocytes sont isolés à partir du tissu adipeux abdominal sous-cutané récupéré lors d'opérations de chirurgies esthétiques (plasties abdominales) réalisées sur des femmes. Les cellules sont isolées à partir du tissu frais. Le tissu adipeux est isolé et dissocié par action d'une collagènase (SIGMA^{TM}, 1 mg/ml, 30 minutes à 37°C, agitation douce). La collagènase digère le tissu conjonctif présent dans le tissu adipeux. Après digestion, les cellules sont filtrées et lavées dans un milieu de culture approprié contenant le milieu MEM sans rouge de phénol, sans glutamine (SIGMA) + 2,2 mg/ml de bicarbonate de sodium (GIBCO) + 50 UI de pénicilline (BIOWHITTAKER^{TM}) + 50 µg/ml de streptomycine (BIOWHITTAKER^{TM}) + 1% (v/v) de L-glutamine (BIOWHITTAKER^{TM}) + 0,5% d'albumine sérique délipidée (SIGMA^{TM}). La suspension d'adipocytes est utilisée immédiatement après sa préparation.

### (ii) Incubation des produits avec les adipocytes

Les produits à tester sont dilués dans le milieu de culture des adipocytes. Ils sont incubés avec les cellules en suspension pendant deux heures à 37°C (250 µl de produit + 250 µl de suspension d'adipocytes).

### (3) - Evaluation des résultats

A l'issue de l'incubation, la lyse cellulaire est contrôlée à vue, par la présence d'une couche lipidique à la surface de la suspension cellulaire. Les milieux sous-nageants sont prélevés. Les acides gras libres sont dosés par spectrophotométrie à l'aide d'un kit commercial, (kit NEFA^{TM} C, WAKO), par référence à une gamme étalon d'acides gras. L'activité lipolytique des produits est évaluée par rapport à un groupe témoin incubé en présence des adipocytes et en absence de produit. La réactivité des adipocytes est systématiquement contrôlée pour la mesure de l'activité lipolytique des produits de référence, la caféine (1,3,7-triméthyl xanthine) et la théophylline (1,3-diméthyl 2,6-dihydroxy purine). On effectue 5 dosages pour chacun des produits testés On a ainsi évalué l'activité lipolytique du LIPACIDE^{TM} C8G qui contient en matière active 100 % de N-octanoyl glycine.

Les résultats des essais, exprimés par la moyenne arithmétique des cinq dosages réalisés pour chacun des produits, sont consignés dans le tableau suivant :

| | Concentration d'incubation (% poids / volume) | Concentration en acides gras libres (µM) | activité lipolytique (par rapport au témoin = 100) |
|---|---|---|---|
| Témoin | - | 11,54 ±2,71 | 100 |
| Caféine | 0,0019 | 15,10 ±4,11 | 131 |
| Théophylline | 0,0019 | 16,53 ± 4,23 | 143 |
| LIPACIDE^{TM} C8G | 0,0001 | 23,83 ± 5,11 | 206 |
| LIPACIDE^{TM} C8G | 0,0005 | 25,30 ± 2,70 | 219 |
| LIPACIDE^{TM} C8G | 0,0010 | 25,75 ± 0,86 | 223 |

Ces résultats font apparaître que, tandis que les composés amincissants de l'état de la technique (la caféine et la théophylline), agissent sur la lipolyse avec un facteur multiplicateur de 1,3 à 1,4 par rapport au témoin, celui selon l'invention c'est à dire comprenant au moins un composé de formule (I) telle que définie précédemment, le fait avec un facteur de 2,06 à 2,23 à des concentrations plus faibles.

### B) - Exemples de formulation cosmétiques

Dans les exemples suivants les proportions sont exprimées en pourcentages pondéraux.

### Exemple 1 : Lait corporel amincissant

| | |
|---|---|
| MONTANOV^{TM} L | 3,00% |
| Phytosqualane | 8,00% |
| Huile d'amandes douces | 2,00 % |
| | |
| Eau | qsp 100 % |
| | |
| SEPIGEL^{TM} 501 | 1,50 % |
| | |
| LIPACIDE^{TM} C8G | 3,00 % |
| | |
| | |
| SEPICIDE^{TM} CI | 0,20 % |
| SEPICIDE^{TM} HB | 0,30 % |
| Parfum | 0,30 % |

### Exemple 2 : Crème anti-relâchement (cible ovale du visage)

| | |
|---|---|
| MONTANOV^{TM} 202 | 3,50 % |
| MONTANOV^{TM} 14 | 1,00 % |
| SEPILIFT^{TM} DPHP | 1,00 % |
| LANOL^{TM} 1688 | 15,00 % |
| Huile de germes de blé | 5, 00 % |
| | |
| Eau | qsp 100% |
| | |
| SIMULGEL^{TM} EG | 1,30 % |
| | |
| LIPACIDE^{TM} C8G | 2,00 % |
| SEPICIDE^{TM} CI | 0,20 % |
| SEPICIDE^{TM} HB | 0,30 % |
| Parfum | 0,10 % |

### Exemple 3 : Atomiseur anti-rondeurs

| | |
|---|---|
| MONTANE^{TM} 60 | 3,30 % |
| MONTANOX^{TM} 60 | 1,70 % |
| Caprilic / capric triglycérides | 6,00 % |
| Isohexadecane | 5,00 % |
| | |
| Magnesium Aluminium Silicate | 1,50 % |
| Eau | qsp 100 % |
| | |
| SIMULGEL^{TM} EG | 1,00 % |
| | |
| | |
| LIPACIDE^{TM} C8G | 2,00 % |
| Centella asiatica / extrait d'hydrocotyl | 1,00 % |
| SEPICIDE^{TM} CI | 0,20 % |
| SEPICIDE^{TM} HB | 0,30 % |
| Parfum | 0,40 % |
| Eau | qsp 100% |

### Exemple 4 : Gel amincissant fraîcheur

| | |
|---|---|
| SEPIGEL^{TM} 305 | 3,50 % |
| Hydroxyéthyl cellulose | 1,00 % |
| Caféine | 5,00 % |
| Menthol | 0,30 % |
| Ethanol | 50,00 % |
| LIPACIDE^{TM} C8G | 3,00 % |
| SEPICIDE^{TM} LD | 1,00 % |
| Parfum | 0,20 % |
| Eau | qsp 100 % |

### Exemple 5 : Fluide corporel minceur

| | |
|---|---|
| SIMULGEL^{TM} NS | 2,50 % |
| Gomme de xanthane | 0,20 % |
| LANOL^{TM} 99 | 5,00 % |
| LIPACIDE^{TM} C8G | 2,00 % |
| Extrait de Ginkgo Biloba | 2,00 % |
| Extrait de cola | 1,00 % |
| Extrait de Ginseng | 0,50% |
| SEPICIDE^{TM} HB | 1,50% |
| Parfum | 0,10% |
| Eau | qsp 100% |

### Exemple 6 : Lotion revitalisante fermeté destinée à être imprégnée sur des lingettes corporelles

| | |
|---|---|
| LIPACIDE^{TM} C8G | 1,50 % |
| Glycérine | 5,00 % |
| Ethanol | 5,00 % |
| Extrait de Ruscus | 3,00 % |
| SEPITONIC^{TM} M3 | 1,00 % |
| | |
| SEPICIDE^{TM} CI | 0,20 % |
| SEPICIDE^{TM} HB | 0,30 % |
| Eau | qsp 100 % |

### Exemple 7 : gel douche amincissant

| | |
|---|---|
| MONTALINE^{TM} C40 | 8,00 % |
| PROTEOL^{TM} OAT | 5,00 % |
| Sodium lauryl sulfate | 9,00 % |
| LIPACIDE^{TM} C8G | 3,00 % |
| Extrait de thé vert | 1,00 % |
| KATHON^{TM} CG | 0,80 % |
| Colorant vert | qs |
| Parfum de thé vert | 1,00 % |
| Acide lactique | qs PH=6,5 |
| Eau | qsp 100% |

### Exemple 8 : Massage désinfiltrant biphasique

| | |
|---|---|
| Huile de café arabica | 1,00 % |
| LANOL^{TM} 189 | 20,00 % |
| LANOL^{TM} 99 | 10,00 % |
| Huile de bourrache | 2,00 % |
| Parfum | 0,10 % |
| | |
| | |
| LIPACIDE^{TM} C8G | 3,00 % |
| Glycérine | 3,00 % |
| Ethanol | 10,00 % |
| Colorant bleu | qs |
| Eau | qsp 100 % |

Les définitions des produits commerciaux utilisés dans les exemples sont les suivantes :
SEPILIFT^{TM} DPHP : (Dénomination INCI : Dipalmitoyl hydroxyproline), commercialisé par la société SEPPIC ;
SEPICIDE^{TM} CI : Imidazoline urée, (agent conservateur) commercialisé par la société SEPPIC ;
SEPICIDE^{TM} HB : Mélange de phénoxyéthanol, de méthylparaben, d'éthylparaben, de propyl-paraben et de butylparaben, (agent conservateur) commercialisé par la société SEPPIC ;
SEPICIDE^{TM} LD : phénoxyéthanol commercialisé par la société SEPPIC ;
KATHON^{TM} CG : (Dénomination INCI : méthyl isothiazolinone / Méthyl chloroisothiazolinone) ;
MONTANE^{TM} 60 : Sorbitan stearate
MONTANOX^{TM} 60 : Polysorbate 60
SIMULGEL^{TM} EG : Latex inverse auto-inversible de copolymère tel que ceux décrits dans la publication internationale WO 99/36445 (dénomination INCI : Sodium acrylate/Sodium acryloyldimethyl taurate copolymer et Isohexadecane et Polysorbate 80 ) commercialisé par la société SEPPIC ;
SIMULGEL^{TM} NS : Latex inverse auto-inversible de copolymère tel que ceux décrits dans la publication internationale WO 99/36445 (dénomination INCI : hydroxyethylacrylate/Sodium acryloyldimethyl taurate copolymer et squalane et Polysorbate 60 ) commercialisé par la société SEPPIC ;
SEPIGEL^{TM} 305 : Latex inverse auto-inversible (dénomination INCI : Polacrylamide / C13-14 Isoparaffin / Laureth-7) ;
SEPIGEL^{TM} 501 : Latex inverse auto-inversible dénomination INCI : C13-14 Isoparaffin/Mineral Oil/Sodium polyacrylate/Polyacrylamide/Polysorbate 85 fin/Mineral Oil/Sodium polyacrylate/Polyacrylamide/Polysorbate 85
LANOL^{TM} 99 : Isononanoate d'isononyle commercialisé par la société SEPPIC ;
LANOL^{TM} 189 : Isostearyl isononanoate
LANOL^{TM} 1688 : Ethylhexanoate de cétéaryle commercialisé par la société SEPPIC ;
SEPITONIC^{TM} M3 : Mélange d'aspartate de magnésium, de gluconate de cuivre et de gluconate de zinc commercialisé par la société SEPPIC ;
MONTALINE^{TM} C40 : Cocamidopropyl bétainamide MEA chloride ;
PROTEOL^{TM} OAT : Sodium Lauroyl oat amino acids
MONTANOV^{TM} 14 : Myristyl alcohol / Myristyl glucoside
MONTANOV^{TM} L : Agent émulsionnant à base d'alcool en C14-C22 et d'alkyl polyglucoside en C12-C20 tel que ceux décrits dans la demande de brevet européen EP 0 995 487 ;
MONTANOV^{TM} 202 est un agent émulsionnant à base d'alcool arachidylique d'alcool béhènylique et d'arachidyl polyglucoside.

## Revendications

1. Utilisation d'un composé de formule (1) : 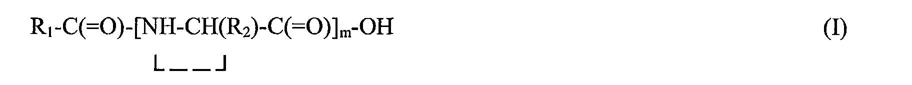 dans laquelle R₁ représente un radical hydrocarboné aliphatique, saturé ou insaturé, linéaire ou ramifié, comportant 7 atomes de carbone, R₂ représente la chaîne caractérisante d'un acide aminé et m est compris entre 1 et 50, ou d'un mélange desdits composés de formule (I), comme actif amincissant, dans une composition contenant un milieu cosmétiquement acceptable.

2. Utilisation telle que définie à la revendication 1, pour laquelle dans la formule (1), R₂ représente la chaîne caractérisante d'un acide aminé choisi parmi la glycine, l'alanine, la sérine, l'acide aspartique, l'acide glutamique, la valine, la thréonine, l'arginine, la lysine, la proline, la leucine, la phénylalanine, l'isoleucine, l'histidine, la tyrosine, le tryptophane, l'asparagine, la glutamine, la cystéine, la cystine, la méthionine, l'hydroxy proline, l'hydroxy lysine, la sarcosine ou l'ornithine.

3. Utilisation telle que définie à la revendication 2, pour laquelle dans la formule (I), R₂ représente la chaîne caractérisante de la glycine.

4. Utilisation telle que définie à l'une des revendications 1 à 3, dans laquelle m est un nombre décimal compris entre 1 et 10 et est de préférence inférieur à 5.

5. Utilisation telle que définie à la revendication 4, dans laquelle m est un nombre décimal inférieur ou égal à 2 et est plus particulièrement inférieur ou égal à 1,4.

6. Utilisation telle que définie à l'une des revendications 1 à 5, dans laquelle m est égal à 1.

7. Procédé de traitement non thérapeutique du corps humain destiné à l'amincir, **caractérisé en ce que** l'on y applique une composition contenant un milieu cosmétiquement acceptable et une quantité efficace d'au moins un composé de formule (I) telle que définie à l'une des revendications 1 à 6.

8. Utilisation d'au moins un composé de formule (I), telle que définie à l'une des revendications 1 à 6, pour préparer un médicament à activité lipolytique, destiné à induire l'amincissement du corps humain.
